# EUROPEAN PATENT APPLICATION

(11) **EP 1 731 031 A1**
(43) Date of publication of application: **13.12.2006**
(21) Application number: 05721614.5
(22) Date of filing: 28.03.2005
(51) Int. Cl.: A01K 67/027, C12N 15/53

(54) **TRANSGENIC FISH WITH INCREASED UNSATURATED FATTY ACID CONTENT**

(30) Priority: 31.03.2004 JP 2004108330
(71) Applicant: Japan Science and Technology Agency, Kawaguchi-shi, Saitama 332-0012 (JP)
(72) Inventor: YOSHIZAKI, Goro, Tokyo 1400002 (JP); TAKEUCHI, Toshiro, Chigasali-shi, Kanagawa 2530082 (JP); SATO, Shuichi, Tachikawa-shi, Tokyo 1900033 (JP); KIRON, Viswanath, Fujisawa-shi, Kanagawa 2510004 (JP); Alimuddin, Tokyo 1080075 (JP)
(74) Representative: Harding, Charles Thomas
(86) International application number: PCT/JP2005/005688
(87) International publication number: WO 2005/094570

(57) **Abstract**

It is intended to utilize a vegetable fat, which is less expensive and can be easily controlled in qualities, thereby largely contributing to the reduction of cost and labor in fish nursery sites and, moreover, enabling the production of fish fry having a high vitality and being tolerant to handling and low temperature without resort to fish oil containing EPA and DHA as a formula feed for fish cultivation. Namely, fish with an increased unsaturated fatty acid content in which a fatty acid desaturase gene is expressed due to the transfer of the fatty acid desaturase gene thereinto. The fatty acid desaturase gene is one or more members selected from among Δ 4 fatty acid desaturase gene, Δ 5 fatty acid desaturase gene and Δ 6 fatty acid desaturase gene, and it is preferable that these genes originate in a freshwater fish. It is preferable that the expression of a fatty acid desaturase gene originating in a freshwater fish is promoted by a β-actin promoter originating in medaka.

SEQUENCE LISTING

<110> Japan Science and Technology Agency

<120> Transgenic fish with the Increase of Unsaturated Fatty Acid

<130> 2005C2529

<160> 10

<170> PatentIn version 3.1

<210> 1
<211> 1692
<212> DNA
<213> Oncorhynchus masou

<400> 1
tgtcattcga tattttcagc aaatgaattg aacaacacat tttgttgtac aacgctgtct 60

ggaaaacatc tcccttccct ctggcagaag aatcgcagtg tgtttcagtt ggacagactg 120

gacagagtgc agccgacaca gcgacggtgt gagtggagca gagagaaccg aggatggggg 180

gcggaggtca gcagacggag tcaagcgagc cggccaaggg tgacggggtt gggcccgatg 240

gagggcgagg tggcagtgca gtctacacct gggaagaggt ccagaagcac tgccacagaa 300

gcgaccagtg gttggtcatc gacaggaagg tctataatat tacccagtgg gcgaagagac 360

acccaggggg catcagggtc atcagtcact ttgctggaga agatgccacg gaagcatttg 420

tcgcattcca tctcgaaccg aattttgtca ggaagtttct gaagccgttg ctgattggag 480

agctggcacc gacagagccc agccaggacc aggggaaaaa tgcagtactg gtgcaggact 540

tccaggccct gcgtgaccgt gtggagagtg agggtctcct ccgtgcccgc cccctcttct 600

tcagcctcta cctgggccac atcctgctac tagaggccct ggctttgggc ctgctctggg 660

tctgggggac cagctggagc ctcacactgc tctgttccct catgctggcc acgtctcagg 720

cccaggctgg ctggctgcag catgactacg gccacctgtc agtctgcaag aaatctggct 780

ggaaccacaa actgcacaag tttgtcattg gacacctaaa gggtgcctct gctaactggt 840

ggaaccatcg tcacttccag caccacgcta agcccaacgt gtttagtaaa gatcctgata 900

tcaactcact gcatgtcttc gtcctgggag acaaacagcc tgtagagtat ggtataaaga 960

agttgaagta catgccctac catcaccaac accagtactt cttcctcgtt ggacctccac 1020

tcatcgttcc agtgtttttc aacatccaga tattccggac catgttttca caacgagact 1080

gggtggatct ggcgtgggcg atgactttct accttcgctt cttctgctgt tactatccct 1140

tctttggttt ctttggctca gtagcattga tcagcttcgt caggtttttg gaaagccact 1200

ggtttgtatg ggtgacccag atgagtcacc ttccgatgga gatggatcac gagagacacc 1260

aggactggct caccatgcag ttgagtgcta cttgcaacat tgaacagtca accttcaacg 1320

actggttcag tggacacctc aactttcaga ttgaacacca tctgtttcct accatgcccc 1380

gtcataacta ccacctggta gctcctctgg ttcgtgcttt gtgtgagaaa catggagttc 1440

cctaccaggt caagactttg cagaaaggca tgactgatgt tgtcaggtca ctgaagaagt 1500

caggggatct gtggctggat gcgtatctcc ataaataaat cccttcctga ctctggacgg 1560

gatttaatcc atcgcagatt aacgacctgc gaacagagag gacatttcct ggatgttttt 1620

gatgataatg atcgctgcaa acattattgt gatataattg ttttaatcct tgtcagcagt 1680

gaatggggat cc 1692

<210> 2
<211> 454
<212> PRT
<213> Oncorhynchus masou

<400> 2

Met Gly Gly Gly Gly Gln Gln Thr Glu Ser Ser Glu Pro Ala Lys Gly
1               5                   10                  15

Asp Gly Val Gly Pro Asp Gly Gly Arg Gly Gly Ser Ala Val Tyr Thr
            20                  25                  30

Trp Glu Glu Val Gln Lys His Cys His Arg Ser Asp Gln Trp Leu Val
        35                  40                  45

Ile Asp Arg Lys Val Tyr Asn Ile Thr Gln Trp Ala Lys Arg His Pro
    50                  55                  60

Gly Gly Ile Arg Val Ile Ser His Phe Ala Gly Glu Asp Ala Thr Glu
65                  70                  75                  80

Ala Phe Val Ala Phe His Leu Glu Pro Asn Phe Val Arg Lys Phe Leu
                85                  90                  95

Lys Pro Leu Leu Ile Gly Glu Leu Ala Pro Thr Glu Pro Ser Gln Asp
            100                 105                 110

Gln Gly Lys Asn Ala Val Leu Val Gln Asp Phe Gln Ala Leu Arg Asp
        115                 120                 125

Arg Val Glu Ser Glu Gly Leu Leu Arg Ala Arg Pro Leu Phe Phe Ser
    130                 135                 140

Leu Tyr Leu Gly His Ile Leu Leu Leu Glu Ala Leu Ala Leu Gly Leu
145                 150                 155                 160

Leu Trp Val Trp Gly Thr Ser Trp Ser Leu Thr Leu Leu Cys Ser Leu
                165                 170                 175

Met Leu Ala Thr Ser Gln Ala Gln Ala Gly Trp Leu Gln His Asp Tyr
            180                 185                 190

Gly His Leu Ser Val Cys Lys Lys Ser Gly Trp Asn His Lys Leu His
        195                 200                 205

Lys Phe Val Ile Gly His Leu Lys Gly Ala Ser Ala Asn Trp Trp Asn
    210                 215                 220

His Arg His Phe Gln His His Ala Lys Pro Asn Val Phe Ser Lys Asp
225                 230                 235                 240

Pro Asp Ile Asn Ser Leu His Val Phe Val Leu Gly Asp Lys Gln Pro
                245                 250                 255

Val Glu Tyr Gly Ile Lys Lys Leu Lys Tyr Met Pro Tyr His His Gln
            260                 265                 270

His Gln Tyr Phe Phe Leu Val Gly Pro Pro Leu Ile Val Pro Val Phe
        275                 280                 285

Phe Asn Ile Gln Ile Phe Arg Thr Met Phe Ser Gln Arg Asp Trp Val
    290                 295                 300

Asp Leu Ala Trp Ala Met Thr Phe Tyr Leu Arg Phe Phe Cys Cys Tyr
305                 310                 315                 320

Tyr Pro Phe Phe Gly Phe Phe Gly Ser Val Ala Leu Ile Ser Phe Val
                325                 330                 335

Arg Phe Leu Glu Ser His Trp Phe Val Trp Val Thr Gln Met Ser His
            340                 345                 350

Leu Pro Met Glu Met Asp His Glu Arg His Gln Asp Trp Leu Thr Met
        355                 360                 365

Gln Leu Ser Ala Thr Cys Asn Ile Glu Gln Ser Thr Phe Asn Asp Trp
    370                 375                 380

Phe Ser Gly His Leu Asn Phe Gln Ile Glu His His Leu Phe Pro Thr
385                    390                    395                    400

Met Pro Arg His Asn Tyr His Leu Val Ala Pro Leu Val Arg Ala Leu
                405                    410                    415

Cys Glu Lys His Gly Val Pro Tyr Gln Val Lys Thr Leu Gln Lys Gly
            420                    425                    430

Met Thr Asp Val Val Arg Ser Leu Lys Lys Ser Gly Asp Leu Trp Leu
        435                    440                    445

Asp Ala Tyr Leu His Lys
    450

<210>   3
<211>   30
<212>   DNA
<213>   Artificial Sequence

<220>
<223>   forward primer

<400>   3
tactccatgg ttcagcaaat gaattgaaca                                        30

<210>   4
<211>   30
<212>   DNA
<213>   Artificial Sequence

<220>
<223> reverse primer

<400> 4
tcgtccatgg ccattcactg ctgacaagga 30

<210> 5
<211> 30
<212> DNA
<213> Artificial Sequence

<220>
<223> forward primer

<400> 5
ttgtcgacgg tctgagtgga gcagagagaa 30

<210> 6
<211> 27
<212> DNA
<213> Artificial Sequence

<220>
<223> reverse primer

<400> 6
atccaggaaa tgtcctctct gttcgca 27

<210> 7
<211> 48
<212> DNA
<213> Artificial Sequence

<220>
<223>  d T 3 RACE-VECT primer

<400>  7
gtaatacgaa taactatagg gcacgcgtgg tcgacggccc gggctggt                48

<210>  8
<211>  26
<212>  DNA
<213>  Artificial Sequence

<220>
<223>  forward primer for desaturation

<400>  8
aggactggct caccatgcag ttgagt                                        26

<210>  9
<211>  20
<212>  DNA
<213>  Artificial Sequence

<220>
<223>  beta-actin forward primer

<400>  9
actacctcat gaagatcctg                                               20

<210>  10
<211>  20
<212>  DNA
<213>  Artificial Sequence

<220>
<223> beta action reverse primer

<400> 10
ttgctgatcc acatctgctg 20

## Description

### Technical Field

The present invention relates to fish with increased unsaturated fatty acid content by transfer of a fatty acid desaturase gene thereinto, and a production method of these fish with increased unsaturated fatty acid content.

### Background Art

The beneficial effect of n - 3 highly unsaturated fatty acids (HUFA), especially eicosapentaenoic acid (EPA, 20:5n - 3) and docosahexaenoic acid (DHA, 22:6n - 3), on human health and development have been extensively studied (for instance, see Non-patent literatures 1, 2 , 3 , 4 , 5). Inhuman, these fatty acids are taken with mainly marine fish or its extract, fish oil. In recent years, however, the availability of raw materials for EPA and DHA production has become increasingly scarce due to a decline in the number of fish captured in the wild (for instance, see Non-patent literature 6). Moreover, these supplies are expected to become critical between 2005 and 2010 (for instance, see Non-patent literature 7). Increase in fish production by means of mariculture production could potentially offset the effects of the decline of EPA and DHA supplies from fish captured in the wild. However, the synthesis of desaturated fatty acid in fish depends on type and its activity of fatty acid metabolic enzymes in the fish. While freshwater fish have enzymes that metabolize linoleic acid to EPA or DHA, marine fish need them for growth and usual development (for instance, see Non-patent literatures 8, 9, 10, 11) although they do not have enzymes that metabolize linoleic acid to EPA or DHA, therefore they take them with feed. EPA and DHA in so-called bluefish are EPA and DHA originating in plankton, and in these fish cultivation, a formula feed highly containing EPA and DHA is needed (for instance, see Non-patent literatures 9, 11). Therefore, an alternative strategy, such as applications of biotechnology, could be utilized to provide an alternative means for producing EPA and DHA in equal or higher levels than that contained in marine fish, compared to when giving fish in fish nursery sites with feed containing EPA and DHA that are disadvantageous in cost performance.

Many studies on these alternative strategies have demonstrated that biosynthesis of EPA in vertebrates, including fish, occurs by the conversion of dietary α-linolenic acid (18:3n - 3) by pathways combining the sequential action of Δ6 and Δ5 desaturation with chain elongation reactions (for instance, see Non-patent literatures 12, 13, 14). Furthermore, it has been described that DHA is synthesized from EPA by the sequential chain elongation to 22:5n - 3 and thence to 24:5n - 3, followed by a L6 - desaturation to 24 : 6n - 3, which is finally retroconverted by peroxisomes via β - oxidation (for instance, see Non-patent literatures 12, 15 , 16, 17) and that a synthesis pathway of a single step for DHA that is directly converted from 22:5n - 3 by a Δ4 desaturation in marine organism (for instance, see Non-patent literature 18).

Recent progresses in transgenic technology have made it possible to produce animals possessing foreign proteins that are beneficial for human health (for instance, see Non-patent literature 19). This technology has also been applied to aquaculture (for instance, see Non-patent literatures 20, 21, 22). It was demonstrated that transgenic fish can be produced by microinjection into the germinal vesicle of oocyte (for instance, see Non-patent literature 23) or into the cytoplasm of a one-cell stage embryo (for instance, see Non-patent literature 24), electroporation using early embryos (for instance, see Non-patent literature 25) or sperm (for instance, see Non-patent literature 26), retroviral infection (for instance, see Non-patent literatures 27, 28) and a particle gun bombardment (for instance, see Non-patent literature 29). All these methods have successfully facilitated the delivery of transgene in certain species of fish, however, a suitable method should be selected for each species (for instance, see Non-patent literature 22). Applications of transgenic technology in fish in order to improve the growth rate, the diseases resistance and adaptation to extreme environmental conditions have been established (for instance, see No - patent literatures 21, 30).

However, there have been very few studies conducted for targeted modification of fish metabolism pathway using transgenic technique. As such study, the potential of gene transfer to compensate for the absence of vitamin C biosynthesis in fish is reported (for instance, see Non-patent literature 31). The method involved introducing the L - guluno - γ - lactone oxidase (rGLO) gene that acted as a catalyst for the terminal reaction for vitamin C. However, no vitamin C production was observed. The improvement of carbohydrate metabolism in rainbow trout by co-injection of a construct containing human glucose transport proteins (hGluT) and rat hexokinase II (rHKII) cDNAs has also been attempted. However, no direct evidence for functionality of rHKII and hGluT1 was observed (for instance, see Non-patent literature 32). In this experiment, a Δ6 desaturase-like gene isolated from yamame salmon (Oncorhynchus masou) was transferred into zebrafish in order to modify fatty acid biosynthesis pathways. The Δ6 desaturase gene was initially targeted due to the fact that this gene is generally considered to be the rate-limiting step in HUFA biosynthesis (for instance, see Non-patent literatures 33, 34).

Non-patent literature 1: Am. J. Clin. Nutr., 54:438-463, 1991
Non-patent literature 2: Nutrition, 16:143-145, 2000
Non-patent literature 3: Nutrition, 16:680-684, 2000
Non-patent literature 4: Prog. Lipid Res, 40:1-94, 2001
Non-patent literature 5: Nutrition, 18:178-188, 2002
Non-patent literature 6: Proc. Nutr. Soc., 58:377-383, 1999
Non-patent literature 7: Aquaculture (In Press), 2002
Non-patent literature 8: J. World Aquacult. Soc., 24:152-161, 1993
Non-patent literature 9: J. Appl. Ichthyol., 11:183-198, 1995
Non-patent literature 10: Aquaculture, 179:217-229, 1999
Non-patent literature 11: Aquaculture, 200:203-222, 2001
Non-patent literature 12: Biochim. Biophys. Acta, 1299:235-244, 1996
Non-patent literature 13: Prog. Lipid Res., 37:73-117, 1998
Non-patent literature 14: Biochim. Biophys. Acta, 1486:219-231, 2000
Non-patent literature 15: Biochem. Physiol, 116B:263-267, 1997
Non-patent literature 16: FEBS Letters, 431:1-6, 1998
Non-patent literature 17: Biochim. Biophys. Acta, 1486:219-231, 2000
Non-patent literature 18: J. Biol. Chem., 276:31561-31566
Non-patent literature 19: Transgenic Res., 9:305-320, 2000
Non-patent literature 20: Biochemistry and molecular biology of fishes, vol.2., pp:207-240, 1993
Non-patent literature 21: Aquaculture, 197:191-204, 2001
Non-patent literature 22: Suisanzoshoku, 49:137-142, 2001
Non-patent literature 23: Cell Differen., 19:237-244, 1986
Non-patent literature 24: Aquaculture, 51:143-150, 1986
Non-patent literature 25: Mol. Mar. Biol. Biotechnol., 1:301-308, 1992
Non-patent literature 26: Aquaculture, 173:297-307, 1999
Non-patent literature 27: Science, 265:666-668, 1994a
Non-patent literature 28: Proc. Natl. Acad. Sci. USA., 93:7777-7782, 1996
Non-patent literature 29: FEBS Letters, 287:118-120, 1991.
Non-patent literature 30: Aquaculture, 204:255-269, 2002
Non-patent literature 31: Biochem. Biophys. Res. Commun., 223:650-653, 1996
Non-patent literature 32: Aquaculture, 173:319-332, 1999a
Non-patent literature 33: Prog. Lipid Res., 20:13-22, 1981.
Non-patent literature 34: J. Biol. Chem., 274:471-477, 1999

As a technology relating to gene expressing fatty acid desaturase, for example, the transformed animals with increased desaturated fatty acid content by transfer of fatty acid desaturase into the animals to transform them and thereby expressing the fatty acid desaturase in the animals (for instance, see Patent document 1), the production method of higher plant cells into which a gene encoding Δ9 desaturase from Anacystis that desaturates Δ9 position of fatty acid bound to lipid is transfered (for instance, see Patent document 2), an isolated Δ5 - fatty acid desaturase from animals and its functional portion (for instance, see Patent document 3) and the method that is the production method of stearidonic acid in seed and the method includes a process to grow plants incorporated with the first DNA construct in the plant genome having functional promoter in plant seed cells, DNA sequence encoding Δ6 - desaturase and functional transcription termination region in plant seed with the transcriptional orientation from 5' to 3' and a process to survive the plant under the condition in which Δ6 - fatty acid desaturase is expressed (for instance, see Patent document 4).

Patent document 1: Unexamined Patent Publication No. 2003-245070
Patent document 2: Unexamined Patent Publication No. 11-332408
Patent document 3: Unexamined Patent Publication No. 14-508932
Patent document 4: Unexamined Patent Publication No. 14-517255

### Disclosure of the Invention

### Problem to be solved by the Invention

Conventionally, fish oil such as EPA and DHA have been needed to be used in the formula feed for marine fish. Problems to be solved by the invention are, by modifying the fatty acid metabolic pathway of fish, to provide fish having a high vitality and being tolerant to handling and low temperature and its production methods, enabling the use of a vegetable fat or animal fat as a formula feed which are less expensive and can be easily controlled in qualities, thereby largely contributing to the reduction of cost and labor in fish nursery sites, and to provide fish containing increased DHA content as a functional food and its production methods.

### Means to solve the Object

As an initial step in the present invention, selection of an appropriate construct that allows high Δ6 desaturase-like gene expression was conducted, since efficient transcription of foreign genes in host individuals is one of the important factors involved in introducing new phenotype encoded by the foreign gene to produce fish with increased unsaturated fatty acid content (Hacket, 1993; Houdebine, 2000). The teleostean zebrafish, Danio rerio (Westerfield, 1995; Gong, 1999), was employed as a model fish, because it offers several advantages such as a relatively short generation time of 2 - 3 months, a large number of eggs, and ease of maintaining. We found that when Δ6-desaturase-like cDNA from masou salmon (Oncorhynchus masou) was transferred to zebrafish under regulation of the medaka (Oryzias latipes) β-actin promoter, it was expressed mainly in muscle and the content of EPA and DHA was increased, thereby confirmed that Δ6-desaturase-like gene was actually the gene having Δ6 desaturase activity, and the invention was completed.

Namely, the present invention relates to (1) transgenic fish with increased unsaturated fatty acid content, wherein a fatty acid desaturase gene is introduced into the transgenic fish and the fatty acid desaturase gene is expressed therein, (2) the transgenic fish according to (1) wherein the fatty acid desaturase gene is connected downstream of β-actin promoter derived from medaka, (3) the transgenic fish according to (1) or (2) wherein the fatty acid desaturase gene is connected upstream of bovine growth hormone polyadenylation sequence, (4) the transgenic fish according to any one of (1) to (3) wherein the fatty acid desaturase gene which consists of any one of the following (A) to (F) sequences;
(A) A nucleic acid sequence (base sequence) encoding a protein consisting of the amino acid sequence shown by SEQ ID NO: 2;
(B) A nucleic acid sequence that consists of amino acid sequence with deletion, substitution or addition of one or more amino acids in the amino acid sequence shown by SEQ ID NO: 2 and that encodes a protein having fatty acid desaturase activity;
(C) A nucleic acid sequence that encodes a protein consisting of amino acid sequence having not less than 60% of homology with the amino acid sequence shown by SEQ ID NO: 2 and having a fatty acid desaturase activity;
(D) The nucleic acid sequence shown by SEQ ID NO: 1;
(E) A nucleic acid sequence that consists of nucleic acid sequence with deletion, substitution or addition of one or more bases in the nucleic acid sequence shown by SEQ ID NO: 1 and that encodes a protein having fatty acid desaturase activity;
(F) A nucleic acid sequence that hybridizes to the nucleic acid sequence shown by SEQ ID NO: 1 under the stringent conditions and that encodes a protein having a fatty acid desaturase activity, (5) Transgenic fish according to any one of (1) to (4) wherein the unsaturated fatty acid is eicosapentaenoic acid (EPA) or docosahexaenoic acid (DHA), (6) transgenic fish according to any one of (1) to (5) wherein the fish are cultured.

### Effect of the Invention

The invention enables to utilize a vegetable fat or animal fat, which is less expensive and can be easily controlled in qualities, thereby largely contributing to the reduction of cost and labor in fish nursery sites and, moreover, enabling the production of fish fry having a high vitality and being tolerant to handling and low temperature without resort to fish oil containing EPA and DHA as a formula feed for fish cultivation. In addition, since fish produced by the invention contain larger content of DHA than usual individuals, fish production having added value as health food can be realized using closed recirculating system for fish culture. Furthermore, not only for direct use in aquaculture, it can be used as a very useful study tool for elucidating the fatty acid metabolic mechanism in fish at a molecular level, thereby largely contributing to the field of art.

### Best Mode of Carrying Out the Invention

Transgenic fish of the present invention is not limited as long as they are fish with increased unsaturated fatty acid content in which a fatty acid desaturase gene is expressed due to the transfer of the fatty acid desaturase gene. As the fatty acid desaturase gene, their origin is not specially limited and includes animals or vegetables. However, fatty acid desaturase genes such as Δ4 fatty acid desaturase gene, Δ5 fatty acid desaturase gene and Δ6 fatty acid desaturase gene are preferred examples, and they can be used alone or in combination. These Δ4 fatty acid desaturase gene, Δ5 fatty acid desaturase gene and Δ6 fatty acid desaturase gene forms unsaturated bond on 4th, 5th and 6th carbon from the carbon on the carboxyl terminus (delta end)of fatty acid, respectively. As these fatty acid desaturase gene, the ones originating in freshwater fish or plankton are preferred because they do not affect normal development and function in fish, and for example, Δ4 fatty acid desaturase gene from microalga (Tonon, T. , Harvey, D. , Larson, T. R., and Graham, I. A. Identification of a very long chain polyunsaturated fatty acid Δ4 - desaturase from the microalga Pavlova lutheri. FEBS Lett. 553, 440-444 (2003).), Δ5 fatty acid desaturase gene from Atlantic salmon (GenBank: AF478472) or Δ6 fatty acid desaturase gene from yamame salmon (GenBank: AB074149, AB070444) are exemplified.

As preferred examples of Δ6 fatty acid desaturase gene, it is not limited as long as it is a gene consisted of any one of following sequences: (A) A nucleic acid sequence encoding the amino acid sequence shown by SEQ ID NO: 2 (amino acid sequence of Δ6 fatty acid desaturase from yamame salmon) ; (B) A nucleic acid sequence that encodes an amino acid sequence with deletion, substitution or addition of one or more amino acids in the amino acid sequence shown by SEQ ID NO: 2 and having a fatty acid desaturase activity; (C) A nucleic acid sequence that encodes an amino acid sequence having not less than 60% of homology with the amino acid sequence shown by SEQ ID NO: 2 and having a fatty acid desaturase activity; (D) The nucleic acid sequence shown by SEQ ID NO: 1 (nucleic acid sequence of Δ6 fatty acid desaturase from yamame salmon); (E) A nucleic acid sequence that consists of a nucleic acid sequence with deletion, substitution or addition of one or more bases in the nucleic acid sequence shown by SEQ ID NO: 1 and that encodes a protein having fatty acid desaturase activity; or (F) A nucleic acid sequence that hybridizes to the sequence shown by SEQ ID NO: 1 under stringent conditions and that encodes a protein having a fatty acid desaturase activity. Here, a "nucleic acid sequence encoding a protein having a fatty acid desaturase activity" refers to nucleic acid sequences encoding proteins involved in any action to form unsaturated fatty acid in the body of fish, however, its specific mechanism of action is not particularly limited.

The "amino acid sequence with deletion, substitution or addition of one or more amino acids" refers to amino aid sequences with any amino acids of for example 1 - 20, preferably 1 - 15, more preferably 1 - 10, more preferably 1 - 5 are deleted, substituted or added. In addition, a "nucleic acid sequence with deletion, substitution or addition of one or more base" refers to nucleic acid sequences with any bases of for example 1 - 20, preferably 1 - 15, more preferably 1 - 10, more preferably 1 - 5 are deleted, substituted or added.
For example, DNA (mutant DNA) consisted of these nucleic acid sequences with one or more bases are deleted, substituted or added may be prepared according to any method known in the art such as chemical synthesis, genetic engineering method and mutagenesis. Specifically, a mutant DNA can be obtained by the methods in which DNA consisted of nucleic acid sequence shown by SEQ ID NO: 1 is contacted with drug having mutagenicity, is radiated with ultraviolet light, or is introduced a mutation using genetic engineering method, and so on. A site - specific mutagenesis, one of the genetic engineering methods, is useful because specific mutation can be introduced to a specific location, and it can be carried out according to a method described in "Molecular Cloning: A laboratory Mannual, 2nd Ed. , Cold Spring Harbor Laboratory, Cold Spring Harbor, NY., 1989" (hereinafter referred to as "Molecular Cloning 2nd Ed.") and Current Protocols in Molecular Biology, Supplement 1-38, John Wiley & Sons (1987-1997). By expressing this mutant DNA using a suitable expression line, a protein consisted of an amino acid sequence with deletion, substitution or addition of one or more amino acids can be obtained.

The "amino acid sequence having not less than 60% of homology with the amino acid sequence shown by SEQ ID NO: 2" is not limited as long as the homology of the sequence with amino acid sequence shown by SEQ ID NO: 2 is not less than 60%, and for example the homology is more than 60%, preferably more than 70%, more preferably more than 80%, furthermore preferably more than 90%, especially preferably more than 95%, most preferably more than 98%.

The "nucleic acid sequence that hybridizes under stringent conditions" refers to nucleic acid sequences obtained by using nucleic acids such as DNA or RNA as a probe, and by colony - hybridization, plaque - hybridization or Southern blot hybridization. Specifically, DNA that can be identified by hybridization using a filter immobilized with DNA or fragment of the DNA originating in colony or plaque in the presence of 0.7-1.0 M NaCl at 65° C, and by washing of a filter using a 0.1-2 × SSC solution (composition of 1 × SSC solution is 150mM sodium chloride, 15mM sodium citrate) under condition of 65° C. Hybridization can be conducted according to the methods described "Molecular Cloning 2nd.Ed.".

For example, for DNA that can hybridize under stringent conditions, DNA having a certain level of homology with the nucleic acid sequence of DNA which is used as a probe. DNA having for example, more than 60%, preferably more than 70%, more preferably more than 80%, furthermore preferably more than 90%, especially preferably more than 90%, most preferably more than 98% of homology is suitably specified.

Methods for obtaining and preparing a gene of the invention are not specially limited. A gene of the interest can be isolated by preparing an appropriate probe or primer according to the amino acid sequence or nucleic acid sequence information shown by SEQ ID NO: 1 or SEQ ID NO: 2 disclosed in the present specification and by screening a cDNA library which is expected to contain the gene, or a gene of the interest can be prepared by chemical synthesis according to a standard method.

Specifically, a gene of the invention can be obtained by selecting a gene of the interest from the cDNA library which has been prepared according to an ordinary method by using an appropriate probe specific to the genes of the invention from yamame salmon from which a gene of the invention has been isolated. The origin of the cDNA can be various cells and tissues originating in the vegetables. Also, all of total RNA separation from these cells or tissues, separation or isolation of mRNA, obtention and its cloning of cDNA can be conducted according to standard method. The screening method of gene of the invention among cDNA library includes methods used by a person skilled in the art such as method described in "Molecular Cloning 2nd Ed."

In addition, mutant genes or homologous genes of the invention that are consisted of a nucleic acid sequence shown by any one of the (B) to (F) can be isolated by screening a homologue of the DNA from other organisms and the like under suitable conditions by using the nucleic acid sequence shown by SEQ ID NO: 1 or a DNA fragment having part of the nucleic acid sequence. Alternatively, it can be prepared using the method for producing a mutant DNA mentioned above.

Unsaturated fatty acids produced by fatty acid desaturase, which is expressed in fish body, varies depending on the type of the fatty acid desaturase used or fatty acid substrates used. However, docosahexaenoic acid (DHA) or eicosapentaenoic acid (EPA) can be suitably exemplified, and LNA (18:3n - 3), OTA (18:4n - 3), ETA (20:4n - 3), DPA (22:5 - 3), TPA (24:5n - 3), THA (24:6n - 3), linoleic acid (18:2n - 6), γ - linolenic acid (18:3n - 6), eicosatrienoic acid (20:3n - 6), arachidonic acid (20:4n - 6) and docosapentaenoic acid (22:5n - 6) are exemplified other than DHA (22:6n - 3) and EPA (20:5n - 3).

As fish with increased unsaturated fatty acid content of the invention, they may originally have fatty acid desaturase gene or may not have it at all to the extent that fatty acid desaturase introduced is expressed and thereby increasing unsaturated fatty acid content in their body. However, fish subjected for aquaculture, fish in which unsaturated fatty acid content is increased selectively in edible portions such as muscle tissues, and fish in which fatty acid desaturase gene is stably expressed to their progeny are preferable. Teleost is preferred as type of fish. Especially, teleost belonging to Cyprinidae, Cichlidae, Salmonidae, Claridae, Siluridae, Ictaluridae are preferable. Specifically, cultured fish such as buri (Seriola quinqueradiata), madai (Pagrus major), hirame (Paralichthys olivaceus), torafugu (Takifugu rubripes), hiramasa (Seriola aureovittata), kurumaebi (Panaeus japonicus), nijimasu (Oncorhyncus mykiss), kanpachi (Seriola dumerili), shimaaji (Pseudocaranx dentex), mahata (Epinephelus septemfasciatus), kuromaguro (Thunnus thynnus), and carp (for example Cyprinus carpio), zebrafish (Danino rerio), African catfish (Clarias gariepinus), tilapia (Oreochronis niloticus), Atlantic salmon (Salmo Salar) and medaka (Oryzias latipes) can be suitably specified.

For construction of an expression vector for introducing the fatty acid desaturase gene into fish, it is preferable to prepare an expression vector in which fatty acid desaturase gene is connected downstream of a promoter which efficiently expresses the fatty acid desaturase gene in fish cells. These promoters may include β-actin promoter, adipocyteP2(aP2) promoter, Mylz2(Danio rerio myosin light polypeptide 2 skeletal muscle mylz2) promoter, UCP promoter, SV40 promoter, cytomegavirus promoter, EF1αpromoter, Metallothionein promoter, heat shock promoter. However, medaka β-actin promoter and mylz2 promoter are especially preferable in regard to expression efficiency. In addition, it is preferable that a polyadenylation sequence such as a bovine growth hormone polyadenylation sequence is connected downstream of the fatty acid desaturase gene. In addition, an intron sequence and enhancer sequence that function to enhance the gene expression, or a terminator sequence that directs termination of transcription may be used as appropriate. Introduction of the constructed expression vector into fish can be conducted by microinjection to oocytes or germ cells, virus vector infection, particle gun bombardment, electroporation.

Transgenic fish of the invention include for the sake of convenience fish germ cells and fish embryo cells, other than adult fish in which the fatty acid desaturase gene is introduced and their progeny.

The followings explain the invention more specifically using Examples, however, the technical scope of the invention is not limited to these Examples.

### Example 1

### [Zebrafish maintenance and diets]

The zebrafish used was AB strain (walker et al., 1999). Fish were spawned and cultured as outlined by Westerfield (1995), with some modifications. Broodstock were raised in a 40-L aquaria under a photoperiod of 14 hours light and 10 hours dark at 28 ± 1° C. Fish were fed on a commercial diet "otohime" (Nisshin Co.) and Artemia (Salt Creek Inc.) nauplii once daily, respectively. There were 4 aquariums for the broodstock, each containing 12 females and 8 males. These aquariums were divided into two groups, one group for spawning at 10: 30am and the other at 14:00pm. This strategy allowed to carry out microinjection twice daily.

### Example 2

### [Cloning of Δ6 desaturase cDNA]

The Δ6-desaturase-like cDNA was isolated from the liver of yamame salmon by PCR with two degenerated primers designed according to the GenBank database (AB070444). The primers used were (5' - TACTCCATGGTTCAGCAAATGAATTGAACA - 3' ; SEQ ID NO: 3) and (5' - TCGTCCATGGCCATTCACTGCTGACAAGGA - 3'; SEQ ID NO: 4) for forward and reverse, respectively, both containing NcoI site (underlined) to facilitate cloning into the expression vector. PCR amplifications were performed under the following conditions: 94° C for 3 min, followed by 30 cycles of 30 sec at 94° C, 30 sec at 62° C, and 1.5 min at 72° C. The amplified product of 1680 bp were then subcloned into pGEM-T Easy vector (Promega).

### Example 3

### [Construction of transgene expression]

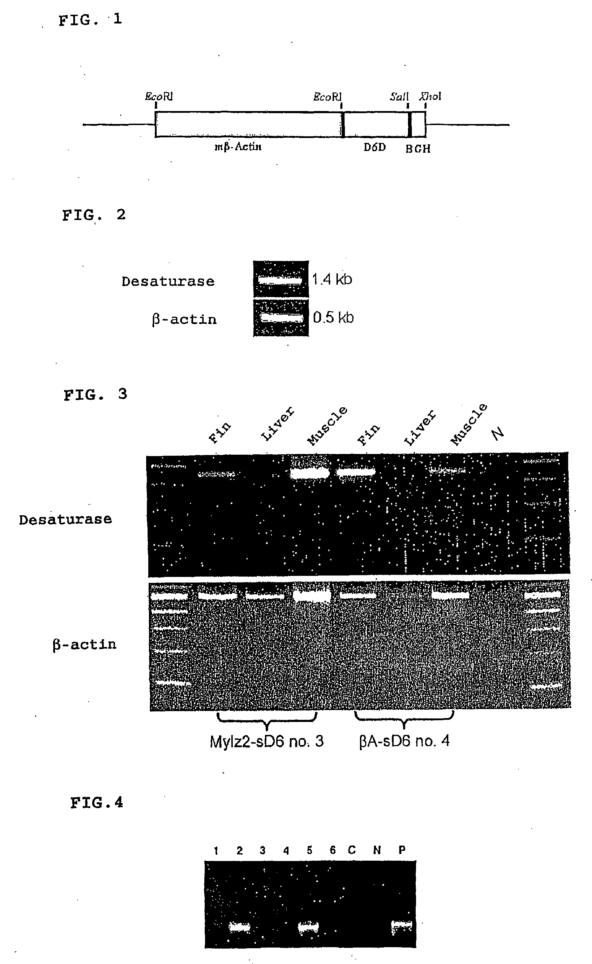

The transgene expression was provided as an 8.5 kb plasmid pActD6 (Figure 1). Briefly, the bovine growth hormone polyadenylation (BGH poly (A)) sequence was removed from the pRc/RSV (Invitrogen) by digestion with XhoI and ligated into pBluescript SK ( + / - ) (Clontech). The 3.7 kb medaka β-actin promoter (mβ-Actin) was modified by PCR from pOBA-109 (Takagi et al., 1994) to provide an EcoRI site for ligation into pBluescript SK ( + / - ) containing BGH poly (A). The 1477 kb Δ6-desaturase-like gene (D6D) of yamame salmon (O. masau) obtained from Example 2 was amplified by PCR with two oligonucleotide primers designed according to the GenBank database (AB070444). Using the forward primer "Sall-desF3" (5' - TTGTCGACGGTCTGAGTGGAGCAGAGAGAA - 3'; SEQ ID NO: 5) containing a Sall recognition site (underlined), and the reverse primer "des-OmaR" (5' - ATCCAGGAAATGTCCTCTCTGTTCGCA - 3'; SEQ ID NO: 6), PCR amplifications were performed under the following conditions: 94° C for 3 min, followed by 30 cycles of 30 sec at 94° C, 30 sec at 62° C, and 1.5 min at 72° C. The PCR amplified products were cloned into the pGEM-T Easy vector (Promega), digested with Sall and then the Δ6-desaturase-like gene fragment was inserted between the medaka β-Actin promoter(mβ-Actin) and the BGH poly (A) sequence to produce the pActD6 construct.

### Example 4

### [Preparation of DNA for microinjection]

Circular DNA obtained from Example 3 was diluted in a solution of 0.1 M KC1/0.125% tetramethyl-rhodamine dextran at a concentration of 30 µg/ml. The DNA solution was then filtered by an Ultrafree-MC Centrifuge Filter Devices 0.22 µm (MILLIPORE, Bedford) and spun at 5,000 rpm in a microcentrifuge at 4° C for 5 min to remove any contaminating particulates. Three to four µL of DNA was transferred onto a clean glass cover and overlaid with a few drops of mineral oil to prevent evaporation.

### Example 5

### [Preparation of microinjection plates]

To make a microinjection plate, a mold was placed onto a 90-mm Petri dish and a warm 3% agarose (prepared in distillated water) of about 30 ml was poured into the plate. After the agarose has solidified at room temperature, the mold was removed to make twelve grooves. These grooves are to support embryos when penetrated by microinjection needle. The used plate was then covered with plastic wrap and stored at 4° C until use.

### Example 6

### [Preparation of the microinjection needle]

Microinjection needles were made with Micropipette Puller (Narishige; Fig. 2A) using a fine-glass microinjection capillary. The tip of the microinjection needle used was about 5 - 8 µm. The needle is attached to the holder and filled with mineral oil from the syringe by turning micromanipulator to the right-hand, through the Teflon tubing, and into the needle.

### Example 7

### [Microinjection set up]

The microinjection system used consists of a stereomicroscope, a micromanipulator prepared in Example 6 for controlling the microinjection needle during the microinjection process, a magnetic stand associated to the micromanipulator (Narishige) for DNA loading, a plastic two-way stopper, Teflon tubing (Narishige), and a microinjection plate. The micromanipulator with a needle holder for DNA loading was attached to the magnetic stand which was firmly fastened to a metal surface. The syringe was filled with 3 ml of mineral oil and was directly connected to the two-way stopper. This stopper was connected to about 40 cm of 1-mm Teflon tubing that was in turn connected to the microinjection needle holder.

### Example 8

### [Collection of eggs and microinjection]

On the day of microinjection, 3 males and 2 females of zebrafish described in example 1 were transferred from their aquarium to the 3-L breeding tank at least 15 minutes before the lamp came on. Eggs were collected in a Petri dish approximately 5 minutes after being laid. The 1-cell-stage embryos were then gently transferred onto the groove of the microinjection plate prepared in Example 5 using a pipette. Blastodisc should be facing the microinjection needle. The DNA solution prepared in Example 4 was microinjected into the cytoplasm of 1-cell-stage embryos on the microinjection plate using the microinjection system prepared in Example 7. Embryos that had been injected with DNA were kept at 28 ± 1° C. Unfertilized and deformed eggs were removed 5 - 6 hours after fertilization. Next day, dead and deformed embryos were removed and healthy embryos were transferred into new Petri dishes.

### Example 9

### [RNA isolation and semi-quantitative RT-PCR]

Samples of twenty embryos are taken at 8 hours post fertilization to isolate mRNA in order to quantify the transcript levels. The total RNA was isolated by ISOGEN (Nippon Gene) according to the manufacturer's instructions. Any traces of DNA were degraded with RQ1 RNase-free Dnase (Promega) 2 U/pg for 30 min at 37° C. After phenol-chloroform extraction and ethanol precipitation, pellets were dissolved in diethylpyrocarbonate (DEPC)-treated water. Single-stranded cDNA was synthesized from 2-3 µg of total RNA with Ready-to-Go You-Prime First-Strand Beads (Amersham Pharmacia Biotech) according to the manufacturer's instructions. In this reaction dT3RACE-VECT primer (5'-GTAATACGAATAACTATAGGGCACGCGTGGTCGACGGCCCGGGCTGG(T) - 3'; SEQ ID NO: 7) was used.

Semi-quantitative RT-PCR was conducted to quantify the transcript levels of foreign desaturase gene. PCR analysis was performed in 20 µl of 10 x Ex Taq Buffer, 200 µM of dNTPs, 0.125U of Ex Taq polymerase (Takara), 2 µl of cDNA as template and 1 pmol of each under the following conditions: 94° C for 3 min, followed by 24 cycles of 30 sec at 94° C, 30 sec at 62° C, and 1.5 min at 72° C. The sequences of the forward primer for desaturase and the reverse primer were as follows: "Omar" (5' - AGGACTGGCTCACCATGCAGTTGAGT - 3'; SEQ ID NO: 8) and the above-mentioned "des-Omar" (SEQ ID NO: 4). The β-actin gene expression was also analyzed as an internal control of equal loading RNA. The forward primer for β-actin gene was (5' - ACTACCTCATGAAGATCCTG - 3'; SEQ ID NO: 9) and the reverse primer was (5' - TTGCTGATCCACATCTGCTG - 3'; SEQ ID NO: 10). Two µl from the reaction was electrophoretically separated using 0.7% agarose gel, stained with ethidium bromide, and photographed under ultraviolet light. Fluorescence intensity of each band was quantified by Densitograph (Atto Co. ltd.). The construct showing the strongest expression was then used to produce stable lines.

The desaturase gene expression of F1 and F2 progeny were also analyzed by the method used for F0. Total RNA was extracted from the fin, liver and muscle of one DNA-positive fish to identify foreign gene expression in F1. The total RNA in F2 generation was extracted from at least twenty 2-day-old larvae, and from muscle and liver of at least 10 adult fish.

### Example 10

### [DNA isolation and PCR amplification]

Identification of transgenic individuals was conducted by PCR with DNA template that had been extracted from caudal fin tissue. The DNA was also extracted from 2-day-old pooled-larvae obtained by crossing the DNA-positive F0 with non-transgenic fish in order to identify the germline transmitters. Isolation of DNA genome was performed using a DNA Isolation Kit (Puregene) according to the manufacturer's instructions. PCR reactions and primers used were the same as the method used for RT-PCR.

### Example 11

### [Production of stable lines]

The DNA-positive F0 were raised to adult fish. The matured F0 was crossed with a non-transgenic zebrafish, and at least twenty 2-day-old larvae from the cross were pooled for screening the germline transmitters. The germline-positive F0 were then used to produce F1 and F2 generations. Production and screening procedures for F1 and F2 were the same as the method used for F0.

### Example 12

### [Fatty acid analysis]

The total lipids were extracted from non-transgenic and transgenic fish containing the masou salmon Δ6-desaturase gene. The non-transgenic and transgenic fish were maintained in the same aquarium, and total fatty acid was extracted from total fish. Fatty acids were separated using a gas chromatography (Shimadzu 14B; Shimadzu) equipped with a hydrogen flame ionization detector and a capillary column of 30 m x 0.32 mm x 0.25 µm (Supelco), and the fatty acid methyl esters (FAMEs) were prepared from fatty acids by the method described previously (references). With the measurement with gas chromatography, fatty acids methyl ester peaks were identified by comparison of their retention times with an appropriate FAME standard (Supelco).

### Example 13

### [Production of stable line; Results]

Transient foreign gene expression was analyzed with cDNA synthesized from RNA extracted from the injected embryos at 8 hours post fertilization. The results that indicate transient expression are shown in Fig 2. Also the results that identified foreign gene expression in F1 extracted from fin, liver and muscle of DNA-positive fish are shown in Fig 3. The results of the experiment conducted with the same conditions except using Mylz2 promoter instead of medaka β-actin promoter are also shown in Fig 3. Fig 3 indicates that foreign gene expression was observed in fin and muscle in both cases using medaka β-actin promoter or Mylz2 promoter.

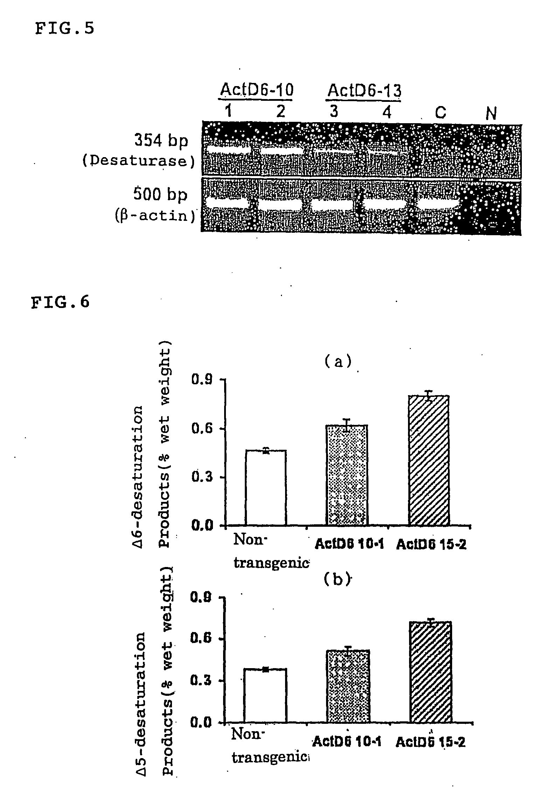

The transgenic individuals were screened by PCR amplification of genomic DNA extracted from fin using primers specific for the masou salmon desaturase gene. The DNA-positive fish was crossed with non-transgenic to screen the germline transmitter's fish and the results are shown in Table 1 and Figure 4. As shown in Table 1, among 400 embryos injected with DNA, 109 were raised and 4 out of the 109 adult fish carried pActD6 gene construct in their germ cells. These fish were then used to produce F1 and F2 generations. The transmission rate of foreign gene into F1 generation was varied between 4.2% and 44.1% as shown in Table 2. These results confirm that transgenic F0 fish were mosaic. The F1 transgenic line which has higher mRNA transcription level analyzed by RT-PCR was used to produce F2 generation. Representative foreign gene expression is shown in Figure 5. The transmission of transgene into F2 generation followed a Mendelian segregation pattern. These results confirmed that all diploid cells of each F1 transgenic line contained transgene.

**[Table 1]**

| Embryos injected | Hatch | Adult stage | DNA-positive fish | Germline transmitter |
|---|---|---|---|---|
| 400 | 178 | 109 | 16 | 4 (3.7%) |

**[Table 2]**

| No. line (F0) I | Inheritance of foreign gene | |
|---|---|---|
| | F1 generation | F2 generation |
| 10 | 39.1% (9/23) | 46.4% (45/97) |
| | | 44.4% (55/124) |
| 12 | 44.1% (15/34) | n.a. |
| 13 | 20.0% (20/100) | n.a. |
| 15 | 4.2% (5/120) | 56.7% (17/30) |
| | | 50.0% (19/30) |
| | | 60.0% (18/30) |

### Example 14

### [Fatty acid composition in transgenic fish]

Fatty acid methyl esters (FAMEs) were prepared both from whole body of non-transgenic and transgenic fish and analyzed by gas chromatography (GC). The n-3 fatty acids compositions of transgenic fish are shown in Table 3. The EPA and DHA production in transgenic fish expressing the masou salmon desaturase gene was 1.4±0.1 fold (1.86±0.03 mg/g vs 1.32±0.05 mg/g) and 2.1±0.2 fold (4.62±0.22 mg/g vs 2.22±0.08 mg/g) higher (p<0.05) than that of non - transgenic counterparts. However, there was highly varied amount of EPA and DHA production between transgenic lines. The amount of EPA and DHA production in edible part was about 75% and 78% to whole body of the fish, respectively.

**[Table 3]**

| Fish | n-3 fatty acids composition | | | | | Total of crude lipid (% wet weight) |
|---|---|---|---|---|---|---|
| | 18:3n-3 | 18:4n-3 | 20:4n-3 | 20:5n-3 | 22:6n-3 | |
| Non-tra nsgenic | 5.30 ± 0.29 (9.39 ± 0.03) | 0.54 ± 0.02 (0.96 ± 0.01) | 0.30 ± 0.00 (0.54 ± 0.03) | 1.32 ± 0.05 (2.34 ± 0.05) | 2.22 ± 0.08 (3.95 ± 0.14) | 5.64 ± 0.30 |
| pActD6 10-1 | 5.91 ± 0.25 (9.58 ± 0.09) | 0.63 ± 0.03 (1.02 ± 0.02) | 0.44 ± 0.04 (0.71 ± 0.05) | 1.70 ± 0.25 (2.74 ± 0.31) | 3.01 ± 0.09 (4.88 ± 0.16) | 6.17 ± 0.22 |
| pActD6 15-2 | 4.30 ± 0.15 (7.77 ± 0.17) | 0.43 ± 0.02 (0.78 ± 0.03) | 0.42 ± 0.01 (0.75 ± 0.01) | 1.86 ± 0.03 (3.37 ± 0.06) | 4.62 ± 0.22 (8.36 ± 0.42) | 5.53 ± 0.13 |

The results shown in Table 3 are means ± SD of triplicate experiments. SD = 0.0 implies an SD < 0.005. Values within parenthesis are percentage of each fatty acid of total fatty acids. Non-transgenic and transgenic fish was maintained in the same aquarium. Total fatty acids were extracted from whole fish, with fatty acid methyl esters having been analyzed and quantified by GC. Numbers at the end of construction name indicate individual transgenic line.

To confirm whether the introduced gene is Δ6- or Δ5-desaturase, the fatty acids except the substrates of desaturation was summed. As shown in Figure 6, the Δ6- and Δ5-desaturation products of transgenic fish was higher (p<0.05) than that of non-transgenic fish. The higher amount of fatty acid products of Δ6-desaturation indicating the introduced gene is attributable to a foreign Δ6-desaturase.

### Brief Description of Drawings

[Fig. 1] It shows a summary of desaturase gene construct inserted to an expression vector used for production of fish with increased unsaturated fatty acid content of the invention.
[Fig. 2] It shows a result of transient expression of desaturase gene by RT - PCR.
[Fig. 3] It shows a result of desaturase gene expression in fin, liver and muscle in cDNA of F1 generation by RT - PCR.
[Fig. 4] It shows representative results of the germline transmitter screening by PCR analysis with DNA template extracted from pooled-larvae. DNA was extracted from about twenty 2-day-old larvae obtained by crossing the DNA-positive F0 with non-transgenic individuals. Lanes 1 through 6; PCR product amplified using genomic DNA samples from transgenic F0 individuals, Lane C; PCR product amplified using non-transgenic fish, Lane N; PCR product amplified without DNA template, Lane P; amplified pActD6 plasmid DNA (0.6 pg).
[Fig.5] It shows a result of detection of transgene expression in different F1 generation by RT - PCR. Total RNA was extracted from caudal fin tissue. Numbers at the end of construction name indicate individual transgenic line.
[Fig. 6] It shows content of desaturated product in fish with increased unsaturated fatty acid content of the invention.

## Claims

1. Transgenic fish with increased unsaturated fatty acid content, wherein a fatty acid desaturase gene is introduced into the transgenic fish and the fatty acid desaturase gene is expressed therein.

2. The transgenic fish according to claim 1, wherein the fatty acid desaturase gene is connected downstream of β-actin promoter derived from medaka.

3. The transgenic fish according to claim 1 or 2, wherein the fatty acid desaturase gene is connected upstream of a bovine growth hormone polyadenylation sequence.

4. The transgenic fish according to any one of claims 1 to 3, wherein the fatty acid desaturase gene which consists of any one of the nucleic acid sequences of following (A) to (F);
(A) Nucleic acid sequence encoding a protein which consists of the amino acid sequence shown by SEQ ID NO: 2;
(B) Nucleic acid sequence that encodes a protein consisting of an amino acid sequence with deletion, substitution or addition of one or more amino acids in the amino acid sequence shown by SEQ ID NO: 2 and having a fatty acid desaturase activity;
(C) Nucleic acid sequence that encodes a protein consisting of an amino acid sequence having not less than 60% of homology with the amino acid sequence shown by SEQ ID NO: 2, and having a fatty acid desaturase activity;
(D) Nucleic acid sequence shown by SEQ ID NO: 1;
(E) Nucleic acid sequence that consists of a nucleic acid sequence with deletion, substitution or addition of one or more bases in the nucleic acid sequence shown by SEQ ID NO: 1 and that encodes a protein having a fatty acid desaturase activity;
(F) Nucleic acid sequence that hybridizes to the nucleic acid sequence shown by SEQ ID NO: 1 under stringent conditions and that encodes a protein having a fatty acid desaturase activity.

5. The transgenic fish according to any one of claims 1 to 4, wherein the unsaturated fatty acid is eicosapentaenoic acid (EPA) or docosahexaenoic acid (DHA).

6. The transgenic fish according to any one of claims 1 to 5, wherein the transgenic fish are cultured fish.
